# EUROPÄISCHE PATENTSCHRIFT

(11) **EP 1 815 812 B1**
(45) Veröffentlichungstag und Bekanntmachung des Hinweises auf die Patenterteilung: **29.07.2009**
(21) Anmeldenummer: 06101271.2
(22) Anmeldetag: 03.02.2006
(51) Int. Cl.: A61B 17/70

(54) **Wirbelsäulenimplantat**
Spinal implant
Implant vertébral

(43) Veröffentlichungstag der Anmeldung: 08.08.2007
(73) Patentinhaber: Spinelab AG, 6304 Zug (CH)
(72) Erfinder: Zehnder Dr., Thomas, 8806, Bäch (CH)
(74) Vertreter: Scheuzger, Beat Otto

(56) Entgegenhaltungen:
- EP-A- 1 527 742
- WO-A-20/04105577
- WO-A-20/05039454
- WO-A-20/06066685
- FR-A- 2 844 180

## Beschreibung

Die vorliegende Erfindung bezieht sich auf ein Wirbelsäulenimplantat, umfassend ein Verbindungselement und mehrere Knochenschrauben die in die Wirbelkörper der Wirbelsäule einschraubbar sind und die mit je einem Kopfteil für die Aufnahme des Verbindungselementes ausgestattet sind mit welchen zwischen Knochenschrauben und Verbindungselement eine feste Verbindung erreichbar ist, und das Verbindungselement aus starren Abschnitten und elastischen Abschnitten zusammensetzbar ist und die starren Abschnitte über Verbindungseinrichtungen mit den elastischen Abschnitten verbindbar sind.

Aus dem Stand der Technik sind derartige Wirbelsäulenimplantate in vielfältiger Weise bekannt. Man unterscheidet hierbei zwischen zwei Systemen, nämlich einem aus einem starren Verbindungselement bestehenden Stabilisierungssystemen, welche eine Versteifung der betroffenen Wirbelkörper zum Ziele haben, und elastischen Systemen, mit welchen eine stützende Stabilisierung der Wirbelkörper erreicht wird, wobei eine gewisse Beweglichkeit zwischen den einzelnen Wirbelkörpern erwünscht und zugelassen wird.

Starre Stabilisierungssystem, die in vielfältiger Weise bekannt sind, haben, wie bereits erwähnt worden ist, das Ziel, dass die problematischen Wirbelkörper eine Versteifung erfahren, so dass ein knöchernes Verwachsen der betroffenen Wirbelkörper erreicht wird. Um eine optimale Stabilisierung erreichen zu können, müssen somit neben den problematischen Wirbelkörpern auch benachbarte gesunde Wirbelkörper mit einbezogen werden, was zur Folge haben kann, dass die Versteifung in einem zu grossen Bereich erfolgt. Zudem ist oft festgestellt worden, dass beim Übergang von stabilisierten zu nicht mehr stabilisierten Wirbelkörpern die Belastung der Wirbelsäule zu gross wird, so dass an dieser Übergangsstelle infolge der zu grossen Belastung der benachbarte Wirbelkörper Schaden nehmen kann.

Wie ebenfalls bereits erwähnt worden ist, werden durch die bekannten elastischen Systeme die Wirbel unterstützend stabilisiert, wobei aber eine gewisse Beweglichkeit zwischen den Wirbelkörpern erwünscht und ermöglicht wird. Dieses System hat aber den Nachteil, dass stark in Mitleidenschaft gezogene Wirbelkörper, bei welchen eine Versteifung erwünscht wäre, mit diesem elastischen System nicht erreicht werden kann, ein knöchernes Verwachsen der betroffenen Wirbelkörper kann nicht erreicht werden.

Aus der FR-A 28 44 180 (Basis für den Oberbegriff des Anspruchs 1) ist ein kombiniertes System bekannt, in welchem elastische und starre Stabilisierungsbereiche eingesetzt werden. Hierbei wird als elastisches Stabilisierungselement eine Spiralfeder-Kunststoff-Kombination verwendet. Dieses elastische Stabilisierungselement wird über eine reine Klemmverbindung mit den Schrauben verbunden.

Die Aufgabe der vorliegenden Erfindung besteht nun darin, ein Wirbelsäulenimplantat zu schaffen, mit welchem es möglich ist, bereichsweise eine starre Stabilisierung der Wirbelkörper zu erreichen, während im angrenzenden Bereich die Wirbelkörper durch ein elastisches System gestützt und stabilisiert werden, ohne dass eine Versteifung erfolgt, und welches optimal und einfach ermöglicht, die starren und die elastischen Elemente miteinander zu verbinden.

Erfindungsgemäss erfolgt die Lösung dieser Aufgabe dadurch, dass die elastischen Abschnitte aus einem biokompatiblen Kunststoff gebildet sind und die Oberfläche mit umlaufenden Rippen und Rillen versehen sind und dass die Verbindungseinrichtungen aus einem aus starrem Material bestehenden U-förmigen Bügel gebildet sind, welche im Grund des Bügels und entlang der Innenseite der Schenkel den Rippen und Rillen der elastischen Abschnitte entsprechende Rillen und Rippen aufweisen.

Mit dieser Ausgestaltung der Erfindung wird ein modulares System geschaffen, bei welchem die Möglichkeit besteht, einander benachbarte Wirbelkörper starr zu stabilisieren, während weitere benachbarte Wirbelkörper elastisch gestützt und stabilisiert werden, wodurch eine eingeschränkte Bewegungsmöglichkeit zugelassen wird. Dadurch hat man verschiedenste Möglichkeiten, die Wirbelsäule bereichsweise elastisch oder starr zu stabilisieren, ohne dass der Eingriff am Patienten aufwendiger würde. Somit kann beispielsweise erreicht werden, dass die Übergangsphase von starr stabilisierten Wirbelkörpern zu den nicht mehr zu stabilisierenden Wirbelkörpern über einen elastisch stabilisierten Bereich schonender zu gestaltet werden kann, im Übergangsbereich vom starr stabilisierten Wirbelkörper zum nicht mehr stabilisierten Wirbelkörper kann somit mindestens eine Teilentlastung der betroffenen Wirbelkörper erreicht werden. Mit diesem modularen Wirbelsäulenimplantat ergeben sich somit an jeden Einzelfall optimal angepasste Lösungen, die einfach zu handhaben sind.

Indem die Verbindungseinrichtungen aus einem aus starren Material bestehenden U-förmigen Bügel gebildet sind, welche im Grund des Bügels und entlang der Innenseite der Schenkel den Rippen und Rillen der elastischen Abschnitte entsprechende Rillen und Rippen aufweisen, erhält man durch das Zusammenwirken der Rippen und Rillen in der Verbindungseinrichtung des elastischen Abschnitts und des U-förmigen Bügels eine feste, formschlüssige Verbindung, eine Pressung des elastischen Abschnitts, die bei einer Klemmverbindung erforderlich wäre und die zu einem Fliessverhalten des elastischen Abschnitts führen könnte, kann somit vermieden werden.

Eine vorteilhafte Ausgestaltung der Erfindung besteht darin, dass die Verbindungseinrichtungen so ausgestaltet sind, dass die jeweils miteinander verbundenen starren Abschnitte und die elastischen Abschnitte im wesentlichen koaxial ausgerichtet sind. Dies ergibt grosse Vorteile und Vereinfachungen beim Einsetzen von derartigen aus starren und elastischen Abschnitten zusammengesetzten Implantaten.

In vorteilhafter Weise sind die starren Abschnitte aus einer metallischen Legierung, insbesondere einer Titanlegierung gebildet, was eine optimale Verträglichkeit bei den mit derartigen Implantaten versorgten Patienten bewirkt.

In vorteilhafter Weise sind die elastischen Abschnitte aus einem biokompatiblen Kunststoff auf Basis von Polyurethan gebildet. Auch dadurch erreicht man eine optimale Verträglichkeit.

In vorteilhafter Weise ist der U-förmige Bügel mit dem eingesetzten elastischen Abschnitt mit einem Verschlussstück verschliessbar, welches zwischen die Bügel einsetzbar und über Einklinkmittel gehalten ist. Dadurch erhält man einen sehr einfachen Vorgang zum Einsetzen und Festhalten des elastischen Abschnitts im U-förmigen Bügel, was sich auf die Dauer des Eingriffs positiv auswirken kann.

Eine weitere vorteilhafte Ausgestaltung der Erfindung besteht darin, dass das Verschlussstück im in den U-förmigen Bügel eingesetzten Zustand auf der dem Abschnitt zugewandten Seite den Rippen und Rillen des elastischen Abschnitts entsprechende Rippen und Rillen aufweist. Dadurch trägt auch das Verschlussstück zur formschlüssigen Verbindung zwischen dem elastischen Abschnitt und dem U-förmigen Bügel in optimaler Weise bei.

In vorteilhafter Weise sind die Verbindungseinrichtungen jeweils an einem Ende eines starren Abschnitts oder am Kopfteil einer Schraube befestigt. Daraus ergibt sich eine optimale Anordnung dieser Verbindungseinrichtungen, das Verbinden der verschiedenen Abschnitte miteinander kann in optimaler Weise erfolgen.

Eine weitere vorteilhafte Ausgestaltung der Erfindung besteht darin, dass die Kopfteile der Schrauben von den Schrauben getrennt sind und einen Aufnahmebereich für die Aufnahme einer Schraube aufweisen, welche Schraube mit dem Kopfteil der Schraube über eine Klemmschraube, mit welcher der starre Abschnitt im Kopfteil der Schraube fixierbar ist, befestigbar ist. Dadurch können zuerst die Schrauben in die Wirbelkörper eingesetzt werden, der Kopfteil, in welchem die Abschnitte gehalten werden können, kann nachträglich bezüglich der Schraube und dem Verbindungselement ausgerichtet werden.

In vorteilhafter Weise weist der Aufnahmebereich des Kopfteils der Schraube für die Aufnahme einer Schraube die Form einer Kugelkalotte auf, und ist der obere Teil der Schraube als Kugelkopf ausgebildet. Dadurch lässt sich der Kopfteil bezüglich der Schraube optimal ausrichten und an die darin aufzunehmenden Abschnitte anpassen.

Ausführungsformen der Erfindung werden nachfolgend anhand der beiliegenden Zeichnung beispielhaft näher erläutert.

Es zeigt:
Fig. 1 in schematischer nicht massstäblicher Darstellung ein in die Wirbelsäule eingesetztes Wirbelsäulenimplantat mit einem Verbindungselement, das aus einem starren Abschnitt und aus einem elastischen Abschnitt zusammengesetzt ist;
Fig. 2 in räumlicher Darstellung das Wirbelsäulenimplantat, wie es in Fig. 1 dargestellt ist;
Fig. 3 in räumlicher Darstellung die Ansicht einer Verbindungseinrichtung;
Fig. 4 in räumlicher Darstellung die Ansicht von weiteren Verbindungseinrichtungen;
Fig. 5a in räumlicher Darstellung eine Verbindungseinrichtung, die am Kopfteil für eine Schraube angebracht ist;
Fig. 5b eine Schnittdarstellung der Verbindungseinrichtung gemäss Fig. 5a;
Fig. 6 eine weitere Anordnung einer Möglichkeit für ein Wirbelsäulenimplantat in räumlicher Darstellung;
Fig. 7 eine Schnittdarstellung einer Schraube mit aufgesetztem Kopfteil, wobei am Kopfteil eine Verbindungseinrichtung angebracht ist; und
Fig. 8 in auseinandergezogener räumlicher Darstellung eine Schraube mit Kopfteil und an diesem Kopfteil angebrachter Verbindungseinrichtung.

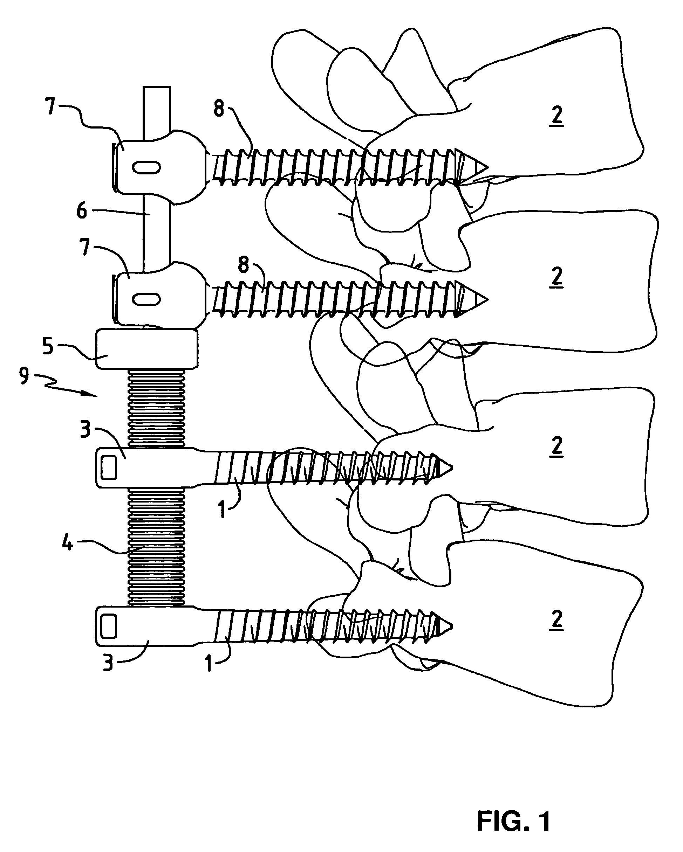

Wie aus Fig. 1 ersichtlich ist, sind erste Schrauben 1 in Wirbelkörper 2 eingeschraubt. Diese ersten Schrauben 1 tragen einen ersten Kopfteil 3, welche zur Aufnahme von einem elastischen Abschnitt 4 ausgebildet sind, wie später noch im Detail beschrieben wird. Der eine Endbereich des elastischen Abschnittes 4 ist in einer Verbindungseinrichtung 5 eingesetzt, welche Verbindungseinrichtung 5, wie ebenfalls später noch im Detail beschrieben wird, an einem starren Abschnitt 6 befestigt ist. Dieser starre Abschnitt 6 ist in zweiten Kopfteilen 7 gehalten, die an zweiten Schrauben 8 angebracht sind, welche ebenfalls in den Wirbelkörpern 2 eingeschraubt sind. Der elastische Abschnitt 4 und der starre Abschnitt 6 bilden zusammen das Verbindungselement 9, mit welchem mit Hilfe der Schrauben 1 und 8 ein Teil einer Wirbelsäule stabilisiert werden kann.

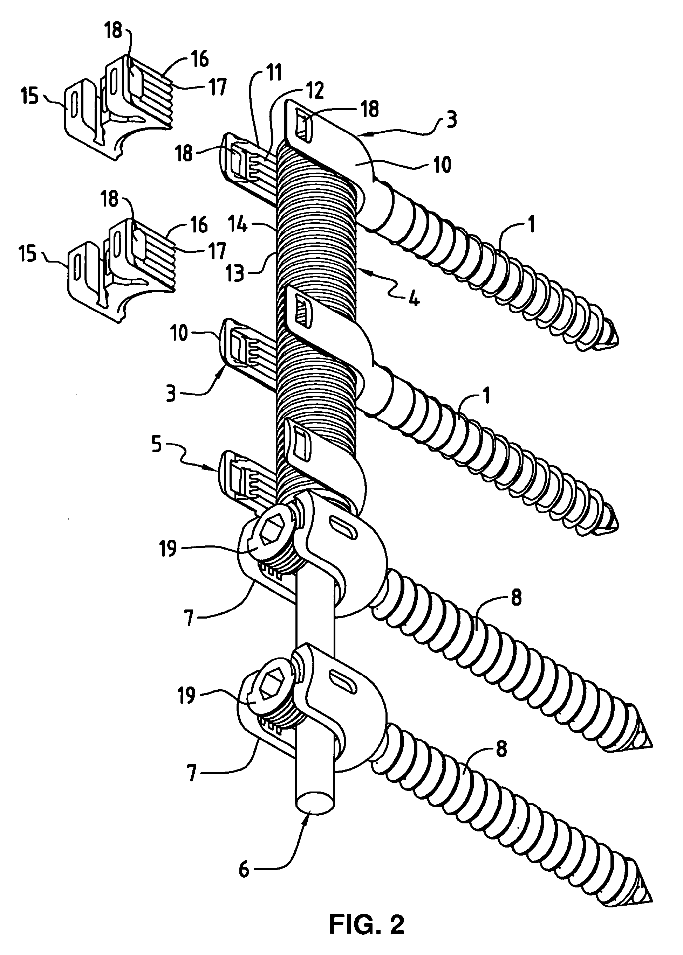

Fig. 2 zeigt erste Schrauben 1 mit ersten Kopfteilen 3 und zweite Schrauben 8 mit zweiten Kopfteilen 7. Die ersten Kopfteile 3 sind jeweils aus einem U-förmigen Bügel 10 gebildet, dessen Innenseite mit Rippen 11 und Rillen 12 versehen sind. Der elastische Abschnitt 4 ist ebenfalls mit umlaufenden Rippen 13 und Rillen 14 versehen. Der elastische Abschnitt 4 wird jeweils in den U-förmigen Bügel 10 eingelegt, wobei die Rippen 11 und Rillen 12 des U-förmigen Bügels 10 jeweils in die Rippen 13 und Rillen 14 des elastischen Abschnittes 4 formschlüssig eingreifen. Der jeweils U-förmige Bügel 10 wird dann mit einem Verschlussstück 15 verschlossen, wobei die Verschlussstücke 15 an der dem elastischen Abschnitt 4 zugewandten Bereich und an den Seiten ebenfalls mit Rippen 16 und Rillen 17 ausgestattet sind, so dass auch diese Verschlussstücke 15 mit dem elastischen Abschnitt 4 und dem U-förmigen Bügel 10 eine formschlüssige Verbindung bilden. Das Verschlussstück 15 kann durch ein Klinkmittel 18 im jeweiligen U-förmigen Bügel 10 gehalten werden. Derartige Schrauben mit dem elastischen Abschnitt und den Verschlussstücken sind beispielsweise in der europäischen Patentanmeldung EP-A-1 527 742 beschrieben.

Die zweiten Kopfteile 7 der zweiten Schrauben 8 sind ebenfalls bügelförmig ausgebildet, so dass ein starrer Abschnitt 6 in diese zweiten Kopfteile 7 der zweiten Schraube 8 eingelegt werden kann. Gehalten wird dieser starre Abschnitt 6 in bekannter Weise über Klemmschrauben 19 in den zweiten Kopfteilen 7 der zweiten Schrauben 8.

Am starren Abschnitt 6 ist die Verbindungseinrichtung 5 befestigt, mit welcher der starre Abschnitt 6 und der elastische Abschnitt 4 miteinander verbunden werden können.

Dieser starre Abschnitt 6 und der elastische Abschnitt 4 könnten auch zu einem vormontierten Verbindungselement 9 zusammengefügt werden und in diesem vormontierten Zustand in die Kopfteile 3 und 7 der bereits in die Wirbelkörper eingeschraubten Schrauben 1 und 8 eingesetzt und fixiert werden.

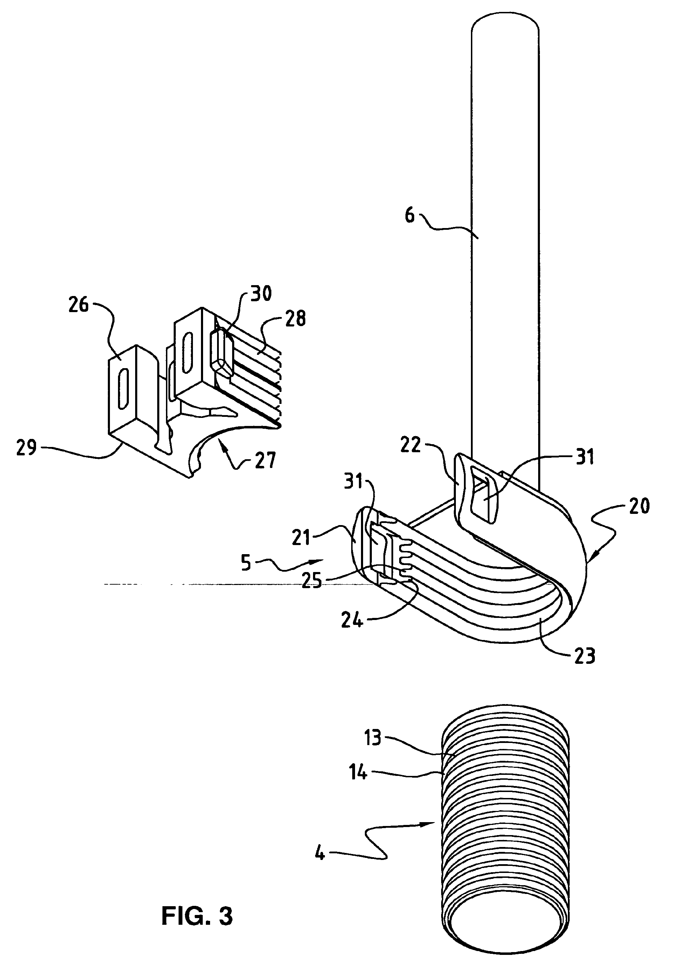

In Fig. 3 ist die Verbindungseinrichtung 5 dargestellt. Am Ende eines starren Abschnittes 6 ist hierzu ein U-förmiger Bügel 20 angeformt. Dieser U-förmige Bügel ist mit zwei Schenkeln 21 und 22 versehen. Im Grund 23 dieses U-förmigen Bügels 20 und entlang der Innenseite der Schenkel 21 und 22 sind Rippen 24 und Rillen 25 angebracht. In diesen U-förmigen Bügel 20 kann dann ein Endbereich des elastischen Abschnittes 4 eingelegt werden, die Rippen 24 und Rillen 25 des U-förmigen Bügels 20 greifen dann in die entsprechenden Rippen 13 und Rillen 14 des elastischen Abschnittes 4 ein, man erhält somit eine formschlüssige Verbindung, wobei der elastische Abschnitt 4 und der starre Abschnitt 6 im verbundenen Zustand koaxial ausgerichtet sind.

Der U-förmige Bügel 20 kann durch ein Verschlussstück 26 verschlossen werden, hierbei ist der U-förmige Bügel 20 gleich ausgebildet wie der U-förmige Bügel 10 der ersten Schraube 1, wie dies in Fig. 2 ersichtlich ist, das Verschlussstück 26 entspricht somit dem Verschlussstück 15 (ebenfalls Fig. 2). Das Verschlussstück 26 weist an der dem elastischen Abschnitt zugewandten Bereich 27 ebenfalls Rippen und Rillen auf, zudem sind entsprechende Rippen und Rillen auch an den Seiten 28 und 29 des Verschlussstückes 26 vorgesehen. Somit ist das Verschlussstück 26 im in den U-förmigen Bügel 20 eingesetzten Zustand über die gerillten Seiten 28 und 29 mit dem U-förmigen Bügel 20 formschlüssig verbunden, ein Formschluss entsteht ebenfalls im Bereich 27, und zwar mit dem elastischen Abschnitt 4. Das Verschlussstück 26 wird über Einklinkmittel 30 im U-förmigen Bügel 20 gehalten.

Der starre Abschnitt 6 und der daran befestigte U-förmige Bügel 20 sind aus einer metallischen Legierung, insbesondere einer Titanlegierung, gebildet. Der elastische Abschnitt besteht aus einem biokompatiblen Kunststoff auf Basis von Polyurethan, wodurch diesem die gewünschte Flexibilität eigen ist, auch das Verschlussstück 26 ist aus dem selben Material gebildet, wodurch zum Einklinken im U-förmigen Bügel 20 die die Einklinkmittel 30 tragenden Bereiche elastisch verformbar sind und die Einklinkmittel 30 in den entsprechenden Ausnehmungen 31 des U-förmigen Bügels 20 einklinken können.

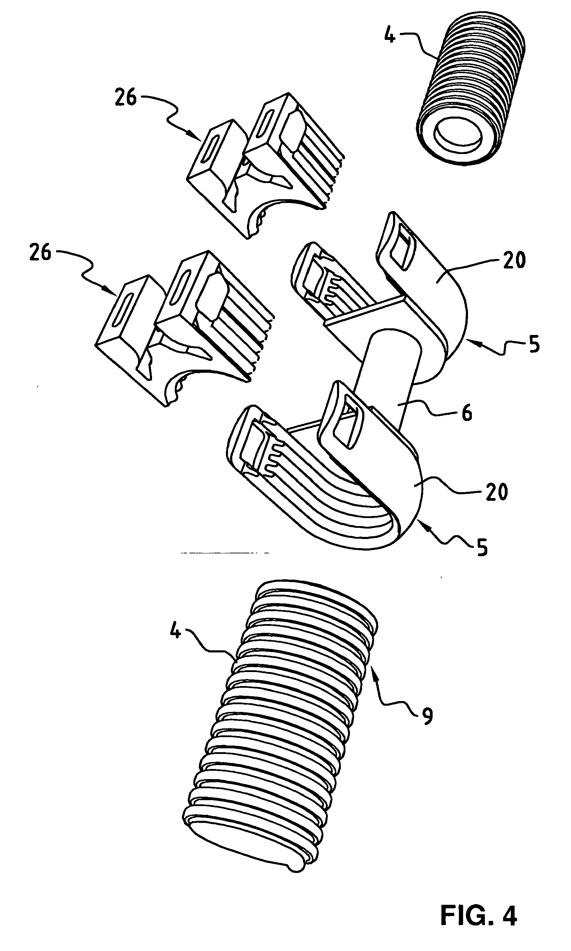

Fig. 4 zeigt einen starren Abschnitt 6, an dessen beiden Endbereichen jeweils eine Verbindungseinrichtung 5 befestigt ist, wie sie zu Fig. 3 beschrieben worden ist. In die U-förmigen Bügel 20 kann jeweils ein elastischer Abschnitt 4 eingesetzt werden und mit dem Verschlussstück 26 gehalten werden. So kann ein im Vergleich mit dem in Fig. 3 dargestellten Verbindungselement 9 unterschiedlich ausgebildetes Verbindungselement 9 erhalten werden, der starre Abschnitt 6 wird durch zweite Schrauben 8 gehalten, während die elastischen Abschnitte 4 durch erste Schrauben 1 (Fig. 2) gehalten werden können. Durch diese Anordnung könnten beispielsweise zwei Wirbelkörper über den starren Abschnitt 6 und die entsprechenden zweiten Schrauben 8 starr stabilisiert werden, die benachbarten Wirbelkörper, in welche die ersten Schrauben 1 eingesetzt werden, können elastisch stabilisiert werden.

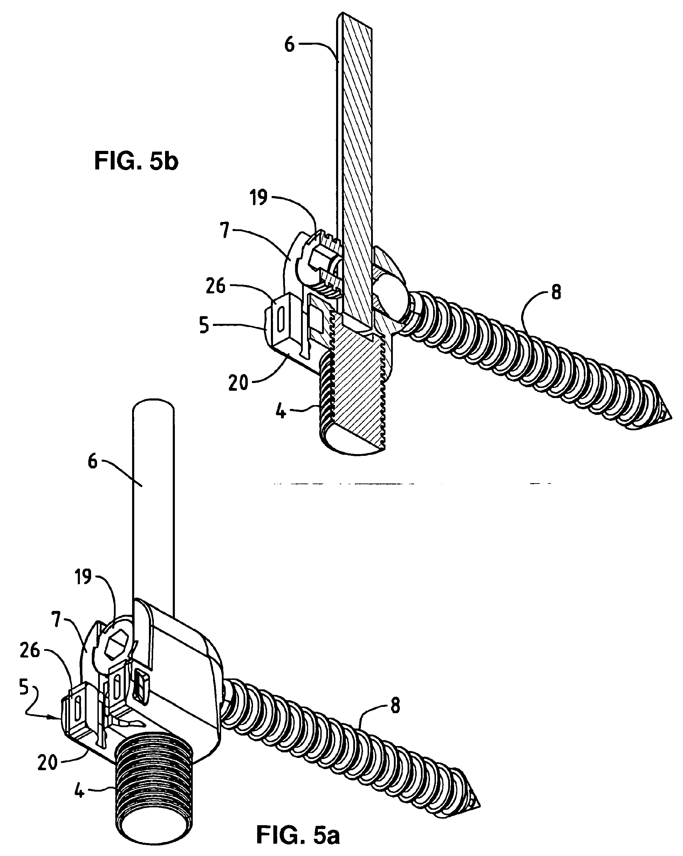

Die Fig. 5a und 5b zeigen eine Verbindungseinrichtung 5, die am zweiten Kopfteil 7 einer zweiten Schraube 8 befestigt ist. Auch hier ist die Verbindungseinrichtung 5 in identischer Weise aufgebaut, wie sie zu Fig. 3 beschrieben worden ist, mit der Ausnahme, dass der U-förmige Bügel 20 nicht am starren Abschnitt 6 befestigt ist, sondern am zweiten Kopfteil 7 der zweiten Schraube 8. Der starre Abschnitt 6 ist, wie bereits erwähnt worden ist, über die Klemmschraube 19 im zweiten Kopfteil 7 der zweiten Schraube 8 gehalten, der elastische Abschnitt 4 ist im U-förmigen Bügel 20 der Verbindungseinrichtung 5 gehalten, wobei dieser U-förmige Bügel 20 wiederum mit einem Verschlussstück 26 verschlossen ist.

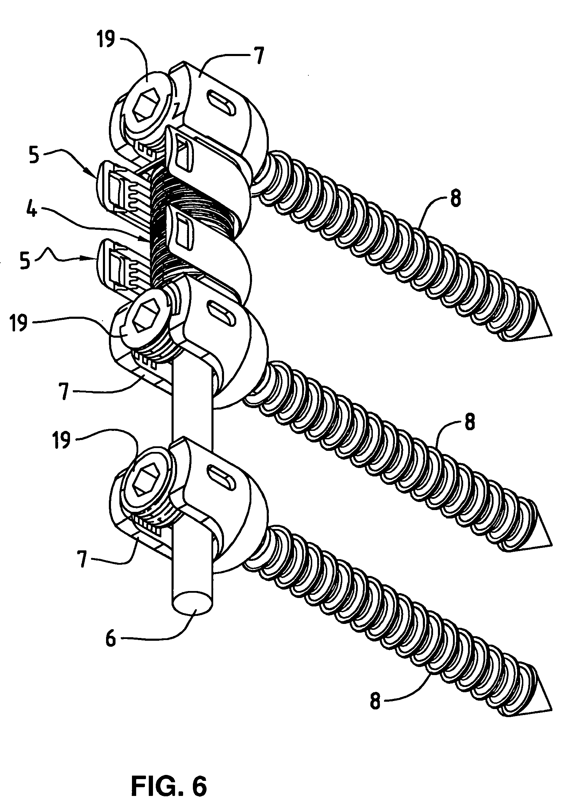

Fig. 6 zeigt in einem Anwendungsbeispiel drei zweite Schrauben 8 mit zweiten Kopfteilen 7. In die zweiten Kopfteile 7 der beiden in Fig. 6 unten dargestellten zweiten Schrauben 8 ist ein starrer Abschnitt 6 eingesetzt, der in den zweiten Kopfteilen 7 durch die entsprechenden Klemmschrauben 19 gehalten ist. Am zweiten Kopfteil 7 der oberen dargestellten zweiten Schraube 8 ist ebenfalls noch ein kurzer starrer Abschnitt 6 eingesetzt, der in dieser Fig. 6 nicht sichtbar ist und durch die Klemmschraube 19 gehalten ist. Die beiden starren Abschnitte 6 sind an den einander zugewandten Enden jeweils mit einer Verbindungseinrichtung 5 ausgestattet, wie diese zu Fig. 3 beschrieben sind. Die beiden unteren dargestellten zweiten Schrauben 8 sind über den starren Abschnitt 6 starr miteinander verbunden und würden die entsprechenden Wirbelkörper fixieren. Die beiden oberen dargestellten zweiten Schrauben 8 sind über einen elastischen Abschnitt 4 miteinander verbunden, die entsprechenden Wirbel würden hierbei elastisch stabilisiert.

Es wäre auch denkbar, anstelle der beiden oberen dargestellten zweiten Kopfteile 7 solche zweite Kopfteile 7 zu verwenden, die mit Verbindungseinrichtungen 5 ausgestattet sind, wie sie in Fig. 5a und 5b dargestellt und entsprechend beschrieben sind.

Das in Fig. 6 von unten nach oben gesehene und dargestellte Verbindungselement, das aus einem starren Abschnitt 6, einem elastischen Abschnitt 4 und wieder mit einem starren Abschnitt 6 zusammengesetzt ist, könnte auch vormontiert werden und in diesem Zustand in die bereits in die Wirbelkörper eingeschraubten zweiten Schrauben im vormontierten Zustand eingesetzt werden.

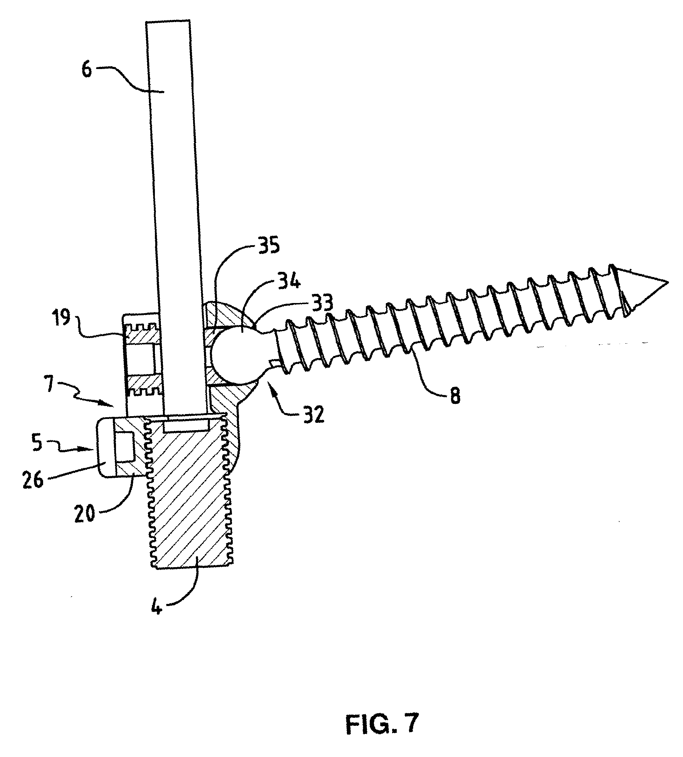

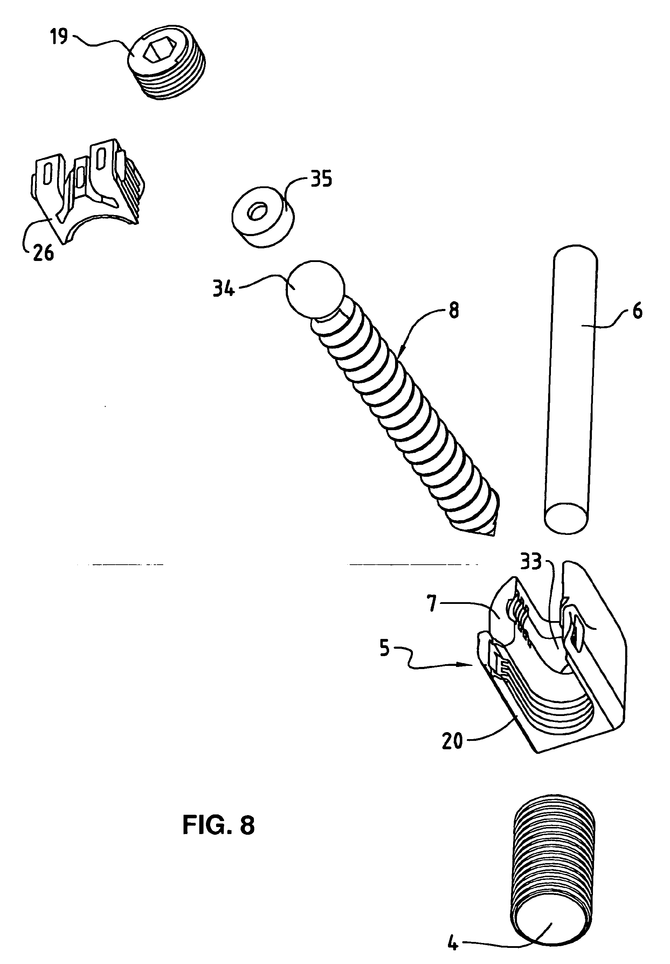

Die beiden Fig. 7 und 8 zeigen eine zweite Schraube 8 mit einem zweiten Kopfteil 7, an welchem Kopfteil 7 ein U-förmiger Bügel 20 einer Verbindungseinrichtung 5 befestigt ist. Die Schraube 8 ist vom Kopfteil 7 getrennt. Der Kopfteil 7 weist einen Aufnahmebereich 32 auf, der die Form einer Kugelkalotte 33 aufweist. Die zweite Schraube 8 ist mit einem Kugelkopf 34 ausgestattet. Die zweite Schraube 8 kann nun so in den zweiten Kopfteil 7 eingesetzt werden, dass der Kugelkopf 34 der zweiten Schraube 8 in die Kugelkalotte 33 des zweiten Kopfteils zu liegen kommt. Auf den Kugelkopf 34 wird dann ein Einsatzstück 35 eingelegt. Nun kann der starre Abschnitt 6 in den zweiten Kopfteil 7 der zweiten Schraube 8 eingelegt werden, über die Klemmschraube 19 wird dann dieser starre Abschnitt 6 gegen das Einsatzstück 35 gepresst, welche Pressung an den Kugelkopf 34 der zweiten Schraube 8 weitergegeben wird und dieser Kugelkopf 34 dann in der Kugelkalotte 33 fixiert wird. Dadurch erhält man einen beweglichen zweiten Kopfteil bezüglich der zweiten Schraube 8, welche im eingesetzten und fixierten Zustand aber gegenseitig keine Bewegung mehr zulassen. Dadurch kann der zweite Kopfteil 7 und somit der starre Abschnitt 6 bezüglich der zweiten Schraube 8 eingestellt und dann fixiert werden. In den U-förmigen Bügel 20 lässt sich dann, wie bereits vorgängig beschrieben worden ist, der elastische Abschnitt 4 einsetzen und über das Verschlussstück 26 fixieren.

Mit diesem erfindungsgemässen Wirbelsäulenimplantat ergeben sich praktisch beliebige Kombinationsmöglichkeiten, so dass ein Verbindungselement aufgebaut werden kann, welches in die entsprechenden Knochenschrauben eingelegt werden kann und welches die gewünschte Stabilisierung auf die einzelnen Wirbelsäulenbereiche ermöglicht.

## Patentansprüche

1. Wirbelsäulenimplantat, umfassend ein Verbindungselement (9) und mehrere Knochenschrauben (1, 8), die in die Wirbelkörper (2) der Wirbelsäule einschraubbar sind und die mit je einem Kopfteil (3, 7) für die Aufnahme des Verbindungselementes (9) ausgestattet sind, mit welchen zwischen Knochenschrauben (1, 8) und Verbindungselement (9) eine feste Verbindung erreichbar ist, und das Verbindungselement (9) aus starren Abschnitten (6) und elastischen Abschnitten (4) zusammensetzbar ist und die starren Abschnitte (6) über Verbindungseinrichtungen (5) mit den elastischen Abschnitten (4) verbindbar sind, **dadurch gekennzeichnet, dass** die elastischen Abschnitte (4) aus einem biokompatiblen Kunststoff gebildet sind und die Oberfläche mit umlaufenden Rippen (13) und Rillen (14) versehen sind und dass die Verbindungseinrichtungen (5) aus einem aus starrem Material bestehenden U-förmigen Bügel (20) gebildet sind in den ein elastischer Abschnitt eingelegt werden kann, welche im Grund (23) des Bügels (20) und entlang der Innenseite der Schenkel (21, 22) den Rippen (13) und Rillen (14) der elastischen Abschnitte (4) entsprechende Rillen (25) und Rippen (24) aufweisen.

2. Wirbelsäulenimplantat nach Anspruch 1, **dadurch gekennzeichnet, dass** die Verbindungseinrichtungen (5) so ausgestaltet sind, dass die jeweils miteinander verbundenen starren Abschnitte (6) und die elastischen Abschnitte (4) im wesentlichen koaxial ausgerichtet sind.

3. Wirbelsäulenimplantat nach Anspruch 1 oder 2, **dadurch gekennzeichnet, dass** die starren Abschnitte (6) aus einer metallischen Legierung, insbesondere einer Titanlegierung, gebildet sind.

4. Wirbelsäulenimplantat nach einem der Ansprüche 1 bis 3, **dadurch gekennzeichnet, dass** die elastischen Abschnitte (4) aus einem biokompatiblen Kunststoff auf Basis von Polyurethan gebildet sind.

5. Wirbelsäulenimplantat nach einem der Ansprüche 1 bis 4, **dadurch gekennzeichnet, dass** der U-förmige Bügel (20) mit dem eingesetzten elastischen Abschnitt (4) mit einem Verschlussstück (26) verschliessbar ist, welches zwischen die Schenkel (21, 22) des Bügel (20) einsetzbar und über Einklinkmittel (30) gehalten ist.

6. Wirbelsäulenimplantat nach Anspruch 5, **dadurch gekennzeichnet, dass** das Verschlussstück (26) im in den U-förmigen Bügel (20) eingesetzten Zustand auf der dem elastischen Abschnitt (4) zugewandten Seite den Rippen (13) und Rillen (14) des elastischen Abschnitts (4) entsprechende Rippen und Rillen aufweist.

7. Wirbelsäulenimplantat nach einem der Ansprüche 1 bis 6, **dadurch gekennzeichnet, dass** die Verbindungseinrichtungen (5) jeweils an einem Ende eines starren Abschnittes (6) befestigt sind.

8. Wirbelsäulenimplantat nach einem der Ansprüche 1 bis 6, **dadurch gekennzeichnet, dass** die Verbindungseinrichtungen (5) jeweils am Kopfteil (7) einer Schraube (8) befestigt sind.

9. Wirbelsäulenimplantat nach einem der Ansprüche 1 bis 8, **dadurch gekennzeichnet, dass** die Kopfteile (7) der Schrauben (8) von den Schrauben (8) getrennt sind und einen Aufnahmebereich (32) für die Aufnahme einer Schraube (8) aufweisen, welche Schraube (8) mit dem Kopfteil (7) der Schraube (8) über eine Klemmschraube (19), mit welcher der starre Abschnitt (6) im Kopfteil (7) der Schraube (8) fixierbar ist, befestigbar ist.

10. Wirbelsäulenimplantat nach Anspruch 9, **dadurch gekennzeichnet, dass** der Aufnahmebereich (32) des Kopfteils (7) der Schraube (8) für die Aufnahme einer Schraube (8) die Form einer Kugelkalotte (33) aufweist und der obere Teil der Schraube (8) als Kugelkopf (34) ausgebildet ist.

## Claims

1. Vertebral column implant, comprising a connecting element (9) and a multiplicity of bone screws (1, 8), which are screwable into the vertebral bodies (2) of the vertebral column and are each provided with a head part (3, 7) for receiving the connecting element (9), with which head parts a firm connection is achievable between bone screws (1, 8) and connecting element (9), and the connecting element (9) is able to be put together from rigid sections (6) and elastic sections (4), and the rigid sections (6) are connectible to the elastic sections (4) via connecting devices (5), **characterised in that** the elastic sections (4) are made of a biocompatible synthetic material and the surfaces are provided with encircling ribs (13) and grooves (14), and **in that** the connecting devices (5) are composed of a U-shaped bow (20) made of a rigid material, in which bow an elastic section can be placed, which connecting devices have at the bottom (23) of the bow (20) and along the inner side of the legs (21, 22) grooves (25) and ribs (24) corresponding to the ribs (13) and grooves (14) of the elastic sections (4).

2. Vertebral column implant according to claim 1, **characterised in that** the connecting devices (5) are designed such that the rigid sections (6), connected together in each case, and the elastic sections (4) are aligned substantially coaxially.

3. Vertebral column implant according to claim 1 or 2, **characterised in that** the rigid sections (6) are made of a metal alloy, in particular a titanium alloy.

4. Vertebral column implant according to one of the claims 1 to 3, **characterised in that** the elastic sections (4) are made of a biocompatible synthetic material, based on polyurethane.

5. Vertebral column implant according to one of the claims 1 to 4, **characterised in that** the U-shaped bow (20) with the inserted elastic section (4) is lockable with a locking piece (26), which is insertable between the legs (21, 22) of the bow (20) and is held via engagement means (30).

6. Vertebral column implant according to claim 5, **characterised in that** the locking piece (26) has, in the state of being inserted in the U-shaped bow (20), ribs and grooves on the side facing the elastic section (4), corresponding to the ribs (13) and grooves (14) of the elastic section (4).

7. Vertebral column implant according to one of the claims 1 to 6, **characterised in that** the connecting devices (5) are each attached at an end of a rigid section (6).

8. Vertebral column implant according to one of the claims 1 to 6, **characterised in that** the connecting devices (5) are each attached at the head part (7) of a screw (8).

9. Vertebral column implant according to one of the claims 1 to 8, **characterised in that** the head parts (7) of the screws (8) are separate from the screws (8), and have a receiving region (32) for receiving a screw (8), which screw (8) with the head part (7) of the screw (8) is attachable via a clamping screw (19), with which the rigid section (6) is able to be fixed in the head part (7) of the screw (8).

10. Vertebral column implant according to claim 9, **characterised in that** the receiving region (32) of the head part (7) of the screw (8) has the shape of a spherical recess (33) for receiving a screw (8), and the upper portion of the screw (8) is designed as a spherical head (34).

## Revendications

1. Implant vertébral, comprenant un élément de liaison (9) et plusieurs vis d'ostéosynthèse (1, 8) qui peuvent être vissées dans les vertèbres (2) de la colonne et qui sont munies d'une partie de tête (3, 7) pour le logement de l'élément de connexion (9) avec lequel il est possible d'établir une connexion fixe entre des vis d'ostéosynthèse (1, 8) et l'élément de connexion (9), et l'élément de connexion (9) se composant de sections rigides (6) et de sections élastiques (4) et les sections rigides (6) pouvant être reliées par des dispositifs de connexion (5) aux sections élastiques (4), **caractérisé en ce que** les sections élastiques (4) se composent d'une matière synthétique biocompatible et que la surface est munie de nervures circulaires (13) et de rainures (14) et **en ce que** les dispositifs de connexion (5) sont formés par des étriers en forme de U se composant d'un matériau rigide, dans lesquels peut être insérée une section élastique, dispositifs qui présentent au fond (23) de l'étrier (20) et le long de la face interne des côtés de U (21, 22) des nervures (25) et des rainures (24) correspondant aux nervures (13) et aux rainures (14) des sections élastiques (4).

2. Implant vertébral selon la revendication 1, **caractérisé en ce que** les dispositifs de liaison (5) sont équipés de sorte que les sections rigides (6) reliées entre elles et les sections élastiques (4) sont orientées sensiblement coaxialement.

3. Implant vertébral selon la revendication 1 ou 2, **caractérisé en ce que** les sections rigides (6) sont formées d'un alliage métallique en particulier d'un alliage de titane.

4. Implant vertébral selon l'une des revendications 1 à 3, **caractérisé en ce que** les sections élastiques (4) sont formées d'une matière synthétique biocompatible à base de polyuréthane.

5. Implant vertébral selon l'une des revendications 1 à 4, **caractérisé en ce que** l'étrier (20) en forme de U avec la section élastique (4) insérée peut être fermé par une pièce de fermeture (26), qui peut être insérée entre les branches (21, 22) de l'étrier (20) et y être maintenue par des moyens d'encliquetage (30).

6. Implant vertébral selon la revendication 5, **caractérisé en ce que** la pièce de fermeture (26) présente dans l'état inséré dans les étriers (20) en forme de U sur la face tournée vers la section élastique (4) des nervures et rainures correspondant aux nervures (13 et aux rainures (14) de la section élastique (4).

7. Implant vertébral selon l'une des revendications 1 à 6, **caractérisé en ce que** les dispositifs de connexion (5) sont fixés respectivement sur une extrémité d'une section rigide (6).

8. Implant vertébral selon l'une des revendications 1 à 6, **caractérisé en ce que** les dispositifs de connexion (5) sont fixés respectivement sur la partie de tête (7) d'une vis (8).

9. Implant vertébral selon l'une des revendications 1 à 8, **caractérisé en ce que** les parties de tête (7) des vis (8) sont séparées par des vis (8) et présentent une zone de réception (32) pour la réception d'une vis (8) et une zone de réception (32) pour la réception d'une vis (8), vis (8) qui peut être fixée par la partie de tête (7) de la vis (8) par une vis de serrage (19) permettant la fixation de la section rigide (6) dans la partie de tête (7) de la vis (8).

10. Implant vertébral selon la revendication 9, **caractérisé en ce que** la zone de réception (32) de la partie de tête (7) de la vis (8) présente la forme d'une calotte sphérique (33) pour le logement d'une vis (8) et la partie supérieure de la vis (8) est réalisée comme une tête sphérique (34).
